# EUROPEAN PATENT APPLICATION

(11) **EP 2 165 731 A1**
(43) Date of publication of application: **24.03.2010**
(21) Application number: 09170765.3
(22) Date of filing: 18.09.2009
(51) Int. Cl.: A61M 39/06

(54) **Detachable and freely rotating hemostasis valve**

(30) Priority: 19.09.2008 US 98502 P
(71) Applicant: Terumo Medical Corporation, Somerset, New Jersey 08873 (US)
(72) Inventor: Trask, Linda E., Delaware 19711 (US); Popejoy, Spencer K.C., Delaware 19711 (US)
(74) Representative: Jenkins, Peter David

(57) **Abstract**

The present invention is directed to a valve hub comprising a hemostasis valve (2) enclosed within a housing, the housing (3) having an upper portion (7) and a lower portion (8) and an access port (6) aligned with the vertical axis of the hemostasis valve; wherein the lower portion of valve hub is engaged within a connector such as a luer fitting, such that the valve hub rotates freely about its vertical axis. The present invention is also directed to medical devices comprising such valves hubs and catheters, as well as methods for assembling such devices and performing medical procedures using such devices.

## Description

### CROSS-REFERENCE TO RELATED APPLICATIONS

The present invention claims the benefit of U.S. Provisional Patent Application No. 61/098,502, filed September 19, 2008, the disclosure of which is hereby incorporated by reference in its entirety.

### FIELD OF THE INVENTION

This invention is related to hemostasis valves and valve hubs for use in combination with devices such as venous or arterial access catheters, catheter introducers, guiding sheaths and introducer sheaths, in the field of endovascular interventions and other medical procedures; as well as medical devices comprising valves, valve hubs and catheters or introducers, and methods of assembling such devices and performing medical procedures on patients.

### BACKGROUND OF THE INVENTION

Introducers and catheters are often used for medical procedures, and are generally designed to provide luminal access to the internal location of a patient's body, *e*.*g*., the vasculature, from a point external to the patient. A typical introducer or catheter may be used for various purposes, including delivering an agent to the body, acting as a conduit removing tissue from within a patient's body, providing electrical stimulus, measuring functions for diagnostic testing, or introducing cameras, probes or the like into a patient's body for examination or treatment.

Typically, an introducer catheter is inserted via one end into the vasculature of a patient, and its proximal end (that is, the end outside of the body) encompasses a valve to minimize blood loss during the procedure. In various uses, a catheter may be provided with a valve on its proximal end to prevent undesired flow of blood through the lumen of a patient's body. For example, when introducers or catheters are first inserted into a lumen of a patient's body such as a vein or an artery, it is often desirable to control the bleeding through the catheter. A valve designed to prevent undesired flow of blood from the vasculature is typically referred to as a hemostasis valve.

Hemostasis valves and other valves are generally attached to the luminal access device at a point outside the body of the patient. The attachment of the valve to a catheter often takes the form of a luer lock fitting. Luer lock fittings generally comprise male and female interlocking tapered fittings, held together with a pressure/twist fit.

In addition, some operations may require the insertion of more than one catheter; thus, the valve hub that contains the valve may comprise more than just the valve, the luer fitting and an access port aligned with the axis of the valve hub. For example, where the medical procedure would benefit from it, a second access port may also be provided; the second access port not being concentrically aligned with the valve hub. The second access port may be in the form of a side port attachment comprising a side arm.

Any feature of the valve hub that is not aligned with the axis thereof thus presents a range of placements for the hub relative to the catheter to which it is attached. For example, a side port attachment comprising a side arm may extend at an angle from the hub, and this angle may be of importance for numerous functional reasons related to the medical procedure. If the hub is fixed with regard to its luer fitting, then a fixed number of relationships may exist between the features of the hemostasis valve, including the side arm, and the catheter to which it is attached.

Hemostasis valves often allow for a user to select the configuration of the hub in relation to the luer fitting prior to attachment to the catheter. Thus, the relationship of the hub to the catheter (and therefore any features attached to the hemostasis valve) may be affected by the unique needs or goals of the particular medical procedure, and thus can be selected by the operator before the medical procedure. However, once the hemostasis valve is fixedly attached to the catheter, rotation of the hub with respect to the luer fitting is no longer possible. The luer connection must therefore be either partially or wholly disengaged before relative rotation of the valve is possible. Thus, if a patient's needs or an operator's needs change in the middle of a procedure, or if the position of the patient or the operating equipment must be moved, the operator has no ability to readjust the valve or catheter position. This is at the very least inconvenient and can at worst lead to complications such as additional blood loss.

Moreover, in various situations the usefulness of the valve/catheter combination in surgical procedures may be limited, as in the case of removal of large pieces of tissue or other large objects from the body of the patient, or in the case where it would be desirable to insert an instrument directly into the catheter, bypassing the valve. In such situations, it would be advantageous to be able to detach the catheter from the luer fitting temporarily, to further enable removal of the tissue or other objects therethrough, while also being able to reattach the valve to the catheter subsequently to continue the procedure.

Therefore, a need exists in the art for improved devices and methods directed to allowing the valve hub to rotate and be configured independently of a luer fitting, and to improving flexibility, adaptability and ease of use during a medical procedure.

### SUMMARY OF THE INVENTION

In certain embodiments, the present invention is directed to a valve hub comprising:
(a) a static hemostasis valve having a vertical axis and an access port aligned with the vertical axis; and
(b) a housing enclosing the static hemostasis valve, the housing including an upper portion and a lower portion;
wherein the lower portion of the valve hub is engaged within the interior of a connector such that the hemostasis valve rotates freely about its vertical axis.

In certain embodiments, the present invention is directed to a medical device comprising:
(a) a catheter having an elongated body;
(b) a connector having a top end and a bottom end; and
(c) a freely rotating valve hub;
wherein the catheter is connected to the connector through the connector's bottom end; and the freely rotating valve hub is connected to the connector though the connector's top end.

In other embodiments, the present invention is directed to a method of assembling a medical device, the method comprising the steps of:
(a) attaching an end of a catheter to a bottom end of a connector; and
(b) inserting a lower portion of a valve hub into a top end of the connector;
wherein the valve hub rotates fully about its vertical axis after step (b).

### BRIEF DESCRIPTION OF THE DRAWINGS

**Fig. 1** shows a perspective view of a valve according to the current art.

**Fig. 2** shows a perspective view of a hemostasis valve according to an embodiment of the present invention.

**Fig. 3** shows a cross-sectional side view of a valve according to the current art.

**Fig. 4** shows a cross-sectional side view of a hemostasis valve according to an embodiment of the present invention.

### DETAILED DESCRIPTION OF THE INVENTION

All references cited in the disclosure of the present invention are hereby incorporated by reference in their entireties. However, in the event of any conflicts between definitions of terms herein and those of any references cited, the present disclosure controls.

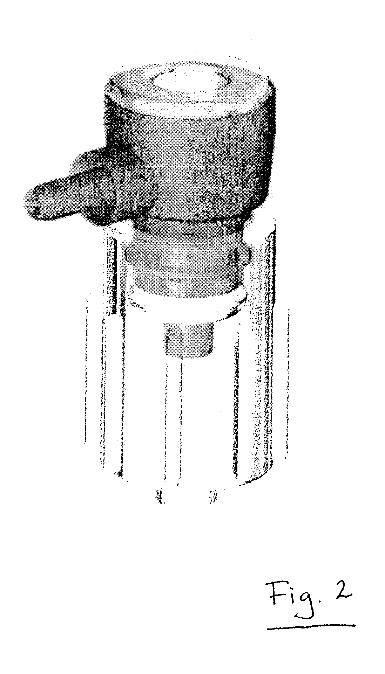

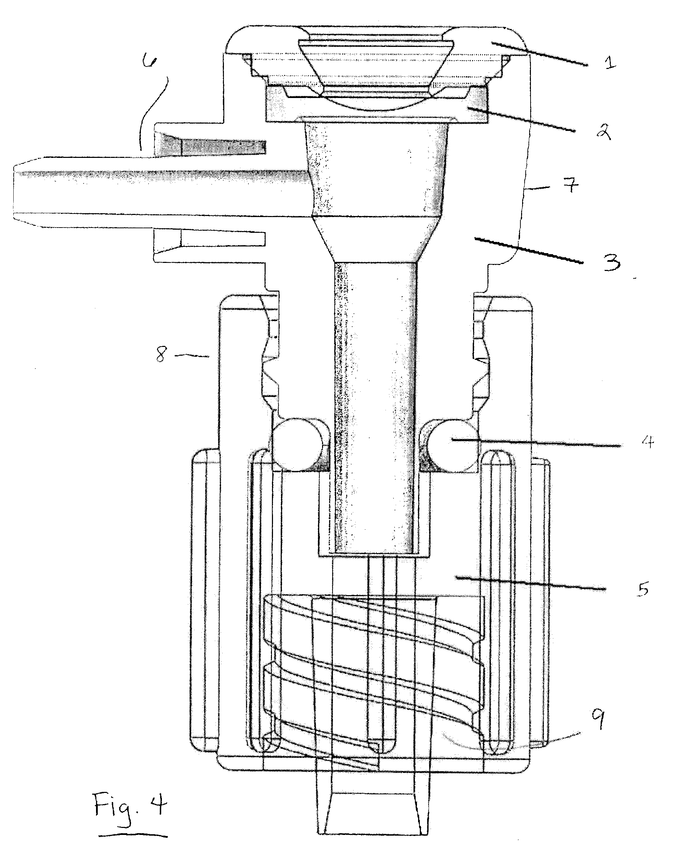

As used herein, the terms "top" and "bottom," and "upper" and "lower" are used, respectively, are used as relative descriptions to refer to directions as shown in the Figures. for example, "top" and "upper" refer to the directions consistent with the top and upper portions of the devices depicted in **Figs. 2** and **4**. However, a skilled artisan will understand that in practice, the devices of the present invention can be turned in any direction while preserving the relative directions denoted herein.

In various embodiments, the present invention is directed to devices comprising a valve hub that is fully and freely rotatable while engaged within connector. In various embodiments, the present invention is directed to devices comprising a valve hub, or valve hub and connector combination, that can be detached from, and reattached to, a catheter in the course of a medical procedure.

In an exemplified version shown in the attached Figures, the housing of the device is a static hemostasis valve enclosed within a cross-cut valve ("CCV") housing. As used herein, the term "valve hub" is used to refer to this static hemostasis valve enclosed within the CCV housing. The valve hubs of the present invention may be made of in whole or in part, materials such as glass, metal, or polymeric materials such as any plastic materials, including polyethylene (PE), polypropylene (PP), high density polyethylene (HDPE), low density polyethylene (LDPE), polyethylene terephthalate (PET), as well as rubber, fiberglass or any other materials that are useful for medical devices, inert and are capable of being sterilized.

In certain embodiments, the CCV housing comprises two slits in a general formation resembling an "x." In certain embodiments, one slit is located on the top surface of the housing, and the other is located on the bottom. Other types of valves may be useful with the devices of the present invention, for example, varying slit patterns. As used herein, the term "static hemostasis valve" distinguishes the valves useful for the embodiments of the present invention from Tuohy-Borst type rotating valves.

In various embodiments, the connector (*e*.*g*., the luer lock fitting) of the present invention is attached to the bottom of the valve hub (the valve hub comprising the valve disposed within the housing). In the embodiment depicted in **Fig. 4****,** the CCV housing encloses the hemostasis valve, and the housing further comprises a cap 1 defining a hole allowing access to the valve within the housing. Thus, in certain embodiments the valve may be pre-enclosed within the housing and then the entire valve hub can be inserted into the connector. Alternatively, in other embodiments, the housing may first be inserted into the connector, and the valve separately inserted in its entirety into the housing via the hole defined by the cap 1. In certain embodiments, the cap is not present during assembly of the valve hub, but after completion of assembly, the cap 1 is then attached to hold the valve in place. In certain embodiments, the cap covers the entire upper portion of the valve hub, and the cap is a large cap that extends over not only the top of the valve hub but also the sides.

The embodiment depicted therein also includes a side arm connector 6. Side arms may be useful for the embodiments of the present invention, as a side arm may provide a path for injection of various agents such as contrast agents or medication (*e*.*g*., anticoagulation drugs, drugs for the treatment of pain or any other adverse conditions that the surgeon may deem necessary), as well as aspiration and a path for removal of substances, *e*.*g*., blood samples, or tissue samples for testing and diagnosis or monitoring of the patient's conditions during the medical procedure. Further useful agents insertable through the side arm are discussed in U.S. Patent No. 5,693,025 to Stevens, hereby incorporated by reference in its entirety.

Nothing in the current art teaches or suggests medical devices directed to hemostasis valves having rotating housing fittings or lock collars. For example, U.S. Patent Nos. 5,989,223 and 5,489, 274 to Chu et al. discuss rotatable valve closures. In these patents, the rotation serves the purpose of opening/loosening or closing/tightening a part of a device to create or close off a defined passage. Thus, the rotation is not free as in the devices and methods of the present invention, because at some point, the part will be fully screwed into the valve, or on the other hand, the part will be fully unscrewed and detached from the valve, therefore bringing the procedure to a halt. It is just this result that the present invention avoids, by permitting constant and free rotation of valve hub and housing fitting or lock collar.

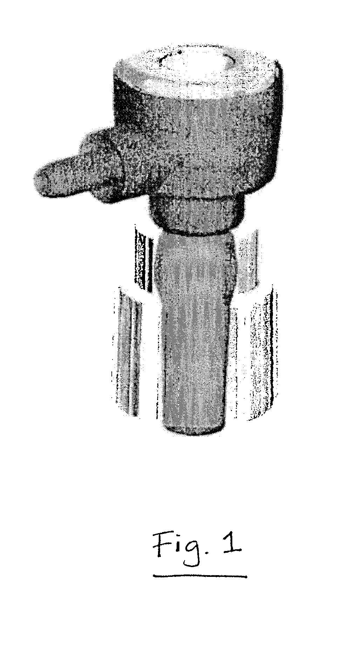

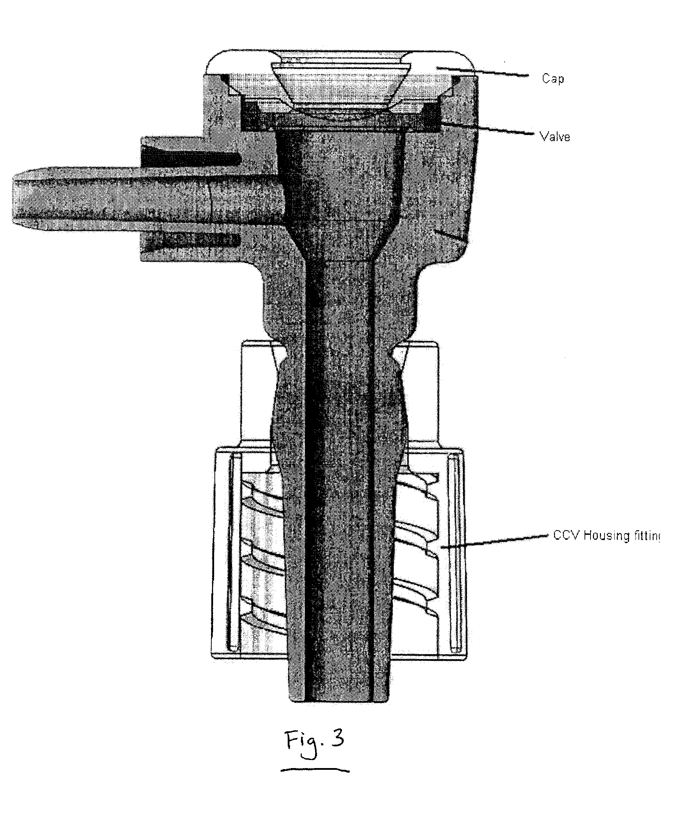

**Fig. 1** and **Fig. 3** depict perspective and cross sectional views, respectively, of a valve currently used in the art. As can be seen from these Figures, currently used valves comprise a lower valve shaft that is completely fixed within a housing, such that its position cannot be altered or rotated while enclosed within the housing. This may provide a tight seal, but this tight seal comes at the expense of flexibility of the valve's configuration. **Fig. 3** depicts the manner in which the lower valve shaft is fixed within the lock collar.

In contrast, the devices and methods of the present invention are **characterized in that** the valve hub (that is, the valve plus its housing) can fully rotate while held within the connector, and that free rotation of the valve hub is possible without detaching the valve hub from its desired location. In certain embodiments, the presence of a gasket can provide further reinforcement of the tight seal, thus preventing leakage while still permitting smooth and free rotation of the valve about its vertical axis. In certain embodiments, the smooth and free rotation is further assisted by the addition of a lubricating composition such as a composition containing oil, silicone, a siloxane or any other composition having a low coefficient of friction.

Thus, the medical devices of the present invention exhibit many advantages over known devices. For example, the ability of the valve hub to rotate fully at all times permits the operator to adjust the relative location of the parts as dictated by the position of other instruments, the needs of the patient, the position of the patient as it may change through the course of the medical procedure, and the like. This is particularly useful where the device includes a side arm. With previously used devices and methods, the presence of a side arm complicates the procedure, as surgeon or operator time and attention must be spent watching the devices to make sure that the connections remain stable even when the valve and/or catheter are moving around. However, with the present invention, the rotatability of the valve hub provides easier manipulation of the parts without fear of disruption of the medical procedure. Rather than having to worry about irreversible detachment of the valve hub or other parts of the device, operator attention can be more effectively focused on the actual procedure.

A cross sectional view of a device of the present invention, including internal parts, is depicted in **Fig. 4**. Included is a valve 2 enclosed in the housing 3 to provide a valve hub, further including a cap 1 on the housing. The housing comprises an upper portion 7 and a lower portion 8. In the embodiment pictured in **Fig. 4****,** the interior of the connector further comprises a gasket 4 that contacts the valve hub at a point along the lower portion of the housing 8 as well as the connector (in this case, a luer lock fitting) 5 enclosing the lower portion of the housing. Also shown is a sideport attachment 6. As can be seen in **Fig. 4**, the gasket provides a further seal and helps to keep the valve hub in place, further permitting it to rotate freely at all times before, during and after the medical procedure, but preventing it from falling out of the connector. As used herein, the term "rotate freely" means that the valve hub can rotate up to 360 degrees (that is, a full circle) about a generally vertical axis, without limitation as to number of times, without substantially altering the position of the valve hub as it is attached to the connector, or otherwise compromising the desirable functions of the devices for their intended purposes in medical procedures.

In certain embodiments, the connector is a luer fitting. The connector has a top end that connects with the lower portion of the valve hub, and a bottom end that can be removeably attached to the end of a catheter. In certain embodiments, the connector's bottom end may further comprise threads 9. The connection to the catheter or introducer is a detachable connection, such that the operator (*e*.*g*., the surgeon, nurse or technician) can detach the catheter from the luer fitting as the need may arise, and reattach it whenever desired to continue the procedure. In various embodiments, the attaching and reattaching steps taking by the operator may be done manually. It is preferable that when attached, the catheter and bottom end of the connector provide a tight seal, but that this seal is readily detached and reattached by the operator as needed, without adverse effect on the patient. In certain embodiments, the attachment may be made through threading either of the connecter or catheter onto each other, by a clamp, by removeable adhesive or adhesive, or by any similar means that are readily and conveniently detachable and reattachable by an operator during the course of a medical procedure.

In certain embodiments, the medical devices of the present invention include a catheter or introducer as commonly used in the art of medical devices. In certain embodiments, the catheter may be a flexible catheter comprising glass, plastic or other polymeric material, may be elongated, and may alternately lie relatively flat during use (that is, be comprised of a soft, flexible and unshapeable material) or be sufficiently rigid that it is shapeable into various curved shapes to facilitate entry of the medical device into a patient's lumen. In other embodiments, the catheter may be completely rigid such that it cannot be manipulated manually to form into other shapes substantially different from its original shape. The catheter may be, in various embodiments, elongated or curved in shape.

In certain embodiments, the present invention is directed to methods of assembling a medical device, comprising the step of inserting a valve hub into a connector. In certain exemplary embodiments, a user first obtains a housing that is not yet attached to a connector. The valve may be inserted into the housing, and the cap put into place to result in the valve hub. Then, the gasket may be slid into the interior of the connector and the valve hub subsequently snapped into the connector. Alternatively, the gasket may be slid onto the lower portion of the housing part of the valve hub, and held in place while both are inserted into the connector. In other embodiments, the housing may already be attached to the connector before the valve is inserted into the housing.

Once the valve hub is engaged within the connector, subsequent detachment of the two may be prevented by, for example, an interference fit near the top of the connector. In other words, the lower portion of the valve hub (as used herein, "lower portion of the valve hub" and "lower portion of the housing" are used interchangeably) may be held in place within the connector within an area of expanded diameter, while a reduced diameter adjacent the top of the connector prevents easy removal of the lower portion of the valve hub therefrom. In other embodiments, a reduced diameter is created with the presence of a ridge or ledge, or multiple tabs, along the interior of the connector. For example, one or more of the lower portion of the valve hub may be made of flexible material that may yield when pushed or downward, thus enabling insertion of the lower portion of the valve hub into the connector past the reduced diameter, and then snap into place once below the area of reduced diameter and fully within the area of expanded diameter, such that the lower portion of the valve hub is engaged within the connector and removal is not easily accomplished.

In various embodiments, the design of the valve hub is such that a user can easily insert the lower portion of the valve hub into the luer fitting, but upon insertion, the expanded diameter portion of the connector holds the lower portion of the valve hub in place and does not permit it to be slid out of the connector in a reverse (upward) direction. In other words, once the lower portion of the valve hub "snaps" into place, its position within the luer fitting is fixed in terms of vertical location; however, it is still capable of rotating continuously and without interruption in a circular, generally horizontal direction about its vertical axis. This difference in width or diameter along the vertical direction of the valve hub can be accomplished with, for example, an indented portion along the length of the valve hub, or ridges or the like. In various embodiments, the valve hub is constructed such that the lower portion of the valve hub is graduated in cross sectional width, *e*.*g*., the lowest end of the valve hub may be (but is not necessarily) narrow enough to pass through the area of reduced diameter with no force, but the cross section toward the middle of the valve will be wider than the area of reduced diameter, and thus held in place once it is pushed downward past the area of reduced diameter with sufficient force.

In certain embodiments, the upper and lower portions of the valve hub may be uniform in diameter; in certain embodiments, the ridge, ledge or multiple tabs along the interior of the connector may be disposed in a direction (*e*.*g*., curved downward) such that insertion of the lower portion of the valve hub past the ridge, ledge or multiple tabs is easily accomplished, but reversing the direction (that is, trying to remove the lower portion of the valve hub) cannot be easily accomplished, as the ridge, ledge or multiple tabs will stay in place due to forces such as gravity or the natural tendency of the ridge, ledge or multiple tabs to remain disposed in a downward direction. In these manners, and in others contemplated by the present invention, the valve hub is held within the connector once the two are attached.

Additionally, in order to preserve a tight seal during use of the medical devices of the present invention, a gasket may be present within the interior of the connector, such that when inserted and in use, the lower portion of the valve hub contacts the gasket. In various embodiments, the gasket may be in the form of an annular ring feature around the entire periphery of the valve, such as that depicted in cross section in **Fig. 4****.** Such annular ring may be an integral part of the interior of the connector or the lower portion of the valve hub, or alternatively may be a separate ring or tabs that are in contact with the lower portion of the valve hub and provide lubrication or a seal to facilitate the medical procedure. In particular, where it is contemplated that the valve hub will be rotating throughout the duration of the procedure, the gasket may serve the advantageous purpose of preventing disruption in the smooth rotation of the various parts, or of maintaining a tight seal within the connector, thus avoiding leakage and consequent disruption of the procedure. The gasket assists with sealing the fit between the CCV housing and connector, preventing ingress or egress of fluid through the valve hub or hemostasis valve. The gasket does not interfere with the free rotation of the CCV valve and connector regardless of what additional elements are attached to the valve or connector.

In certain embodiments, the present invention is directed to methods of performing a medical procedure on a patient, the method comprising the steps of:
(a) removeably attaching a first end of a catheter to the bottom end of a connector;
   wherein the top end of the connector engages a freely rotating valve hub in accordance with the embodiments set forth in the present disclosure;
(b) inserting a second end of the catheter to the interior of a body of a patient; and
(c) rotating the valve hub freely about its vertical axis while changing the position of the catheter. In certain embodiments, the methods may further comprise the steps of:
(d) removing the first end of the catheter from the bottom end of the connector;
(e) reattaching the first end of the catheter to the bottom end of the connector; and
(f) continuing the medical procedure

An important aspect of the interference fit is that free rotation of the connector (such as the luer lock fitting) and the valve hub is allowed without regard to whether the connector is engaged or disengaged from any other structure. Once the valve hub is fully engaged within the connector, the valve hub may freely rotate about the axis formed by the lower portion of the valve hub disposed within the interior of the connector.

Thus, the valve hub may rotate independently of the connector, *e*.*g*., luer lock fitting, regardless of whether or not the connector is engaged with a luminal access device such as a catheter. The valve hub may therefore be oriented in any relationship to the luminal access device and have the orientation altered in any manner at any time.

As discussed herein, the devices and methods of the present invention present many distinct advantages over devices known in the art. As in most medical procedures, the process of inserting catheters and other luminal access devices into a patient's body, as well as frequent pulling out of the same and exchanging devices, often leads to increased handling and thus increased likelihood of "bumping" into such objects. In the case of valves known in the art, where a valve is attached via a screwing mechanism, accidental bumping can lead to unscrewing of various parts, resulting in leakage, inconvenience and possible danger.

Moreover, if a medical device has a certain desired position inside a patient's body, it can be difficult to move. Providing an additional degree of freedom (that is, the ability for the valve hub to rotate freely) makes the process simpler and more convenient. Additionally, in certain embodiments of the present invention, the ability to removeably attach the catheter to the connector may give the operator additional options, such as, for example, when a larger device must be inserted into the catheter, *e*.*g*., stent grafts (which tend to be larger and may be a tight fit through a valve) or material such as tissue from within the patient's body and the like must be removed quickly. In either of those cases, it is a simple matter to detach the catheter end from the bottom end of the connector, perform the additional insertion or removal step, and reattach the catheter end to the bottom end of the connector, thus continuing with the procedure.

All embodiments described herein are illustrative and in no way limit the scope of the invention, and the invention may be embodied in other forms not explicitly described here, without departing from the spirit thereof.

## Claims

1. A valve hub comprising:
(a) a static hemostasis valve having a vertical axis and an access port aligned with the vertical axis; and
(b) a housing enclosing the static hemostasis valve, the housing including an upper portion and a lower portion;
wherein the lower portion of the valve hub is engaged within the interior of a connector such that the hemostasis valve rotates freely about its vertical axis.

2. The valve hub of claim 1, wherein the connector is a luer lock fitting.

3. The valve hub of claim 2, further comprising a gasket disposed on the outer surface of the housing and in contact with both the housing and interior of the connector.

4. The valve hub of claim 1, wherein the gasket is removeable from the housing.

5. The valve hub of claim 1, wherein the housing is a cross cut valve (CCV) housing.

6. The valve hub of claim 1, further comprising a side port attachment, the side port attachment comprising a second access port that is not concentrically aligned with the access port of the hemostasis valve.

7. The valve hub of claim 1, wherein the valve hub comprises, in whole or in part, a material chosen from glass, metal or polymeric material.

8. The valve of claim 1, wherein the connector has a top end and a bottom end, wherein the lower portion of the valve hub is held within the connector through the connector's top end, and
wherein a catheter or introducer is removeably attached to the connector through the connector's bottom end.

9. The valve of claim 8, wherein the connector comprises threads on its bottom end for connection to a catheter or introducer.

10. A medical device comprising:
(a) a catheter having an elongated body;
(b) a connector having a top end and a bottom end; and
(c) a freely rotating valve hub;
wherein the catheter is connected to the connector through the connector's bottom end; and the freely rotating valve hub is connected to the connector though the connector's top end.

11. The catheter of claim 10, further comprising a side port attachment extending from the valve hub.

12. The medical device of claim 10, further comprising a gasket in contact with both the valve hub and the connector.

13. A method of assembling a medical device, the method comprising the steps of:
(a) attaching an end of a catheter to a bottom end of a connector; and
(b) inserting a lower portion of a valve hub into a top end of the connector;
wherein the valve hub rotates fully about its vertical axis after step (b).

14. A method of performing a medical procedure on a patient, the method comprising the steps of:
(a) removeably attaching a first end of a catheter to the bottom end of a connector;
wherein the top end of the connector engages a freely rotating valve hub in accordance with claim 1;
(b) inserting a second end of the catheter to the interior of a body of a patient; and
(c) rotating the valve hub freely about its vertical axis while changing the position of the catheter.

15. The method of claim 14, further comprising the step of:
(d) removing the first end of the catheter from the bottom end of the connector;
(e) reattaching the first end of the catheter to the bottom end of the connector; and
(f) continuing the medical procedure.
